# EUROPÄISCHE PATENTSCHRIFT

(11) **EP 1 516 061 B1**
(45) Veröffentlichungstag und Bekanntmachung des Hinweises auf die Patenterteilung: **06.08.2008**
(21) Anmeldenummer: 03740154.4
(22) Anmeldetag: 28.05.2003
(51) Int. Cl.: C12Q 1/00

(54) **VERFAHREN ZUR IDENTIFIZIERUNG VON AGONISTEN ODER ANTAGONISTEN FÜR DEN GPR45LIKE/GPR63 REZEPTOR**
METHOD FOR IDENTIFYING AGONISTS OR ANTAGONISTS FOR THE GPR45-LIKE/GPR63 RECEPTOR
PROCEDE POUR IDENTIFIER DES AGONISTES OU DES ANTAGONISTES DU RECEPTEUR DE TYPE GPR45/GPR63

(30) Priorität: 08.06.2002 DE 10225651
(43) Veröffentlichungstag der Anmeldung: 23.03.2005
(73) Patentinhaber: Sanofi-Aventis Deutschland GmbH, 65929 Frankfurt am Main (DE)
(72) Erfinder: KOSTENIS, Evi, 36323 Grebenau (DE); GASSENHUBER, Johann, 65185 Wiesbaden (DE)
(86) Internationale Anmeldenummer: PCT/EP2003/005600
(87) Internationale Veröffentlichungsnummer: WO 2003/104480

(56) Entgegenhaltungen:
- WO-A-01/07606
- WO-A-02/34781
- WO-A-99/67383
- US-A- 5 854 004
- NIEDERNBERG ANKE ET AL: "Sphingosine 1-phosphate and dioleoylphosphatidic acid are low affinity agonists for the orphan receptor GPR63." CELLULAR SIGNALLING, Bd. 15, Nr. 4, 20. April 2003 (2003-04-20), Seiten 435-446, XP002251164 ISSN: 0898-6568
- AN S ET AL: "SIGNALING MECHANISMS AND MOLECULAR CHARACTERISTICS OF G PROTEIN-COUPLED RECEPTORS FOR LYSOPHOSPHATIDIC ACID AND SPHINGOSINE 1-PHOSPHATE" JOURNAL OF CELLULAR BIOCHEMISTRY, WILEY-LISS INC, US, Bd. 30/31, 1998, Seiten 147-157, XP000939318 ISSN: 0730-2312

### SEQUENZPROTOKOLL

<110> Aventis Pharma Deutschland GmbH
<120> Verfahren zur Identifizierung von Agonisten oder Antagonisten für den GPR45 like/GPR63 Rezeptor
<130> DEAV2002/0033
<140>
   <141>
<160> 12
<170> PatentIn Ver. 2.1
<210> 1
   <211> 1260
   <212> DNA
   <213> Homo sapiens
<400> 1
   atggtcttct cggcagtgtt gactgcgttc cataccggga catccaacac aacatttgtc 60
   gtgtatgaaa acacctacat gaatattaca ctccctccac cattccagca tcctgacctc 120
   agtccattgc ttagatatag ttttgaaacc atggctccca ctggtttgag ttccttgacc 180
   gtgaatagta cagctgtgcc cacaacacca gcagcattta agagcctaaa cttgcctctt 240
   cagatcaccc tttctgctat aatgatattc attctgtttg tgtcttttct tgggaacttg 300
   gttgtttgcc tcatggttta ccaaaaagct gccatgaggt ctgcaattaa catcctcctt 360
   gccagcctag cttttgcaga catgttgctt gcagtgctga acatgccctt tgccctggta 420
   actattctta ctacccgatg gatttttggg aaattcttct gtagggtatc tgctatgttt 480
   ttctggttat ttgtgataga aggagtagcc atcctgctca tcattagcat agataggttc 540
   cttattatag tccagaggca ggataagcta aacccatata gagctaaggt tctgattgca 600
   gtttcttggg caacttcctt ttgtgtagct tttcctttag ccgtaggaaa ccccgacctg 660
   cagatacctt cccgagctcc ccagtgtgtg tttgggtaca caaccaatcc aggctaccag 720
   gcttatgtga ttttgatttc tctcatttct ttcttcatac ccttcctggt aatactgtac 780
   tcatttatgg gcatactcaa cacccttcgg cacaatgcct tgaggatcca tagctaccct 840
   gaaggtatat gcctcagcca ggccagcaaa ctgggtctca tgagtctgca gagacctttc 900
   cagatgagca ttgacatggg ctttaaaaca cgtgccttca ccactatttt gattctcttt 960
   gctgtcttca ttgtctgctg ggccccattc accacttaca gccttgtggc aacattcagt 1020
   aagcactttt actatcagca caactttttt gagattagca cctggctact gtggctctgc 1080
   tacctcaagt ctgcattgaa tccgctgatc tactactgga ggattaagaa attccatgat 1140
   gcttgcctgg acatgatgcc taagtccttc aagtttttgc cgcagctccc tggtcacaca 1200
   aagcgacgga tacgtcctag tgctgtctat gtgtgtgggg aacatcggac ggtggtgtga 1260
<210> 2
   <211> 419
   <212> PRT
   <213> Homo sapiens
<400> 2
   Met Val Phe Ser Ala Val Leu Thr Ala Phe His Thr Gly Thr Ser Asn
    1               5                   10                  15
   Thr Thr Phe Val Val Tyr Glu Asn Thr Tyr Met Asn Ile Thr Leu Pro
               20                  25                  30
   Pro Pro Phe Gln His Pro Asp Leu Ser Pro Leu Leu Arg Tyr Ser Phe
           35                  40                  45
   Glu Thr Met Ala Pro Thr Gly Leu Ser Ser Leu Thr Val Asn Ser Thr
       50                  55                  60
   Ala Val Pro Thr Thr Pro Ala Ala Phe Lys Ser Leu Asn Leu Pro Leu
   65                  70                  75                  80
   Gln Ile Thr Leu Ser Ala Ile Met Ile Phe Ile Leu Phe Val Ser Phe
                   85                  90                  95
   Leu Gly Asn Leu Val Val Cys Leu Met Val Tyr Gln Lys Ala Ala Met
               100                 105                 110
   Arg Ser Ala Ile Asn Ile Leu Leu Ala Ser Leu Ala Phe Ala Asp Met
           115                 120                 125
   Leu Leu Ala Val Leu Asn Met Pro Phe Ala Leu Val Thr Ile Leu Thr
       130                 135                 140
   Thr Arg Trp Ile Phe Gly Lys Phe Phe Cys Arg Val Ser Ala Met Phe
   145                 150                 155                 160
   Phe Trp Leu Phe Val Ile Glu Gly Val Ala Ile Leu Leu Ile Ile Ser
                   165                 170                 175
   Ile Asp Arg Phe Leu Ile Ile Val Gln Arg Gln Asp Lys Leu Asn Pro
               180                 185                 190
   Tyr Arg Ala Lys Val Leu Ile Ala Val Ser Trp Ala Thr Ser Phe Cys
           195                 200                 205
   Val Ala Phe Pro Leu Ala Val Gly Asn Pro Asp Leu Gln Ile Pro Ser
       210                 215                 220
   Arg Ala Pro Gln Cys Val Phe Gly Tyr Thr Thr Asn Pro Gly Tyr Gln
   225                 230                 235                 240
   Ala Tyr Val Ile Leu Ile Ser Leu Ile Ser Phe Phe Ile Pro Phe Leu
                   245                 250                 255
   Val Ile Leu Tyr Ser Phe Met Gly Ile Leu Asn Thr Leu Arg His Asn
               260                 265                 270
   Ala Leu Arg Ile His Ser Tyr Pro Glu Gly Ile Cys Leu Ser Gln Ala
           275                 280                 285
   Ser Lys Leu Gly Leu Met Ser Leu Gln Arg Pro Phe Gln Met Ser Ile
       290                 295                 300
   Asp Met Gly Phe Lys Thr Arg Ala Phe Thr Thr Ile Leu Ile Leu Phe
   305                 310                 315                 320
   Ala Val Phe Ile Val Cys Trp Ala Pro Phe Thr Thr Tyr Ser Leu Val
                   325                 330                 335
   Ala Thr Phe Ser Lys His Phe Tyr Tyr Gln His Asn Phe Phe Glu Ile
               340                 345                 350
   Ser Thr Trp Leu Leu Trp Leu Cys Tyr Leu Lys Ser Ala Leu Asn Pro
           355                 360                 365
   Leu Ile Tyr Tyr Trp Arg Ile Lys Lys Phe His Asp Ala Cys Leu Asp
       370                 375                 380
   Met Met Pro Lys Ser Phe Lys Phe Leu Pro Gln Leu Pro Gly His Thr
   385                 390                 395                 400
   Lys Arg Arg Ile Arg Pro Ser Ala Val Tyr Val Cys Gly Glu His Arg
                   405                 410                 415
   Thr Val Val
<210> 3
   <211> 42
   <212> DNA
   <213> Homo sapiens
<400> 3
   ccgccgaagc ttgccatggt cttctcggca gtgttgactg cg 42
<210> 4
   <211> 36
   <212> DNA
   <213> Homo sapiens
<400> 4
   gccggcgaat tctcacacca ccgtccgatg ttcccc 36
<210> 5
   <211> 24
   <212> DNA
   <213> Homo sapiens
<400> 5
   cccactggtt tgagttcctt gacc 24
<210> 6
   <211> 24
   <212> DNA
   <213> Homo sapiens
<400> 6
   ggtagcctgg attggttgtg tacc 24
<210> 7
   <211> 18
   <212> DNA
   <213> Homo sapiens
<400> 7
   ctcaacaccc ttcggcac 18
<210> 8
   <211> 20
   <212> DNA
   <213> Homo sapiens
<400> 8
   ggcctggctg aggcatatac 20
<210> 9
   <211> 28
   <212> DNA
   <213> Homo sapiens
<400> 9
   tgccttgagg atccatagct accctgaa 28
<210> 10
   <211> 20
   <212> DNA
   <213> Homo sapiens
<400> 10
   tgcctggaca tgatgcctaa 20
<210> 11
   <211> 19
   <212> DNA
   <213> Homo sapiens
<400> 11
   tccgtcgctt tgtgtgacc 19
<210> 12
   <211> 25
   <212> DNA
   <213> Homo sapiens
<400> 12
   tccttcaagt ttttgccgca gctcc 25

## Beschreibung

Vorliegende Erfindung betrifft ein Verfahren zur Identifizierung einer Verbindung, welche die Aktivität des G Protein gekoppelten Rezeptors GPR45 like/GPR63 verändert.

G Protein gekoppelte Rezeptoren vermitteln extrazelluläre Signale wie beispielsweise von Hormonen, Neurotransmittern, Licht oder Geruchsstoffen über G Proteine ins Zellinnere, wobei über eine intrazelluläre Signalkaskade unterschiedliche Effekte ausgelöst werden können. G Proteine bestehen normalerweise aus drei verschiedenen Untereinheiten (alpha, beta, gamma). Man kennt verschiedene heterodimere G-Proteine, die sich in Rezeptorspezifität und Wirkung unterscheiden. Die G Proteine werden durch GTP aktiviert. Ein bekanntes G Protein ist das Transducin aus dem Sehprozess.

G Protein gekoppelte Rezeptoren (GPCR) spielen eine wichtige Rolle bei einer Vielzahl von physiologischen Prozessen. Sie stellen eine der größten bekannten Proteinfamilien dar. Zur Zeit wird geschätzt, daß etwa 1000 Gene des menschlichen Genoms für diese Rezeptorklasse kodieren. GPCR sind Membranproteine mit 7 transmembranen α-Helices. Eine Vielzahl von Arzneimitteln entfaltet ihre Wirkung über GPCRs.

GPCRs sind insbesondere bei der Signalverarbeitung und Steuerung des Organismus beteiligt und spielen deshalb eine übergeordnete Rolle bei der Aufrechterhaltung der Funktion des intakten Organismus.

Die Bindung eines extrazellulären Liganden führt zu einer Konformationsänderung des betreffenden GPCR. Die Konformationsänderung schafft die Voraussetzung für die Interaktion mit dem jeweils zugeordneten G-Protein. Das G-Protein wiederum löst eine für den entsprechenden Zelltyp charakteristische intrazelluläre Signalkaskade aus. Kennzeichnend für die intrazellulären Signalkaskaden sind die sogenannten "second messengers". Darunter sind niedermolekulare Verbindungen wie beispielsweise cAMP (cyclisches Adenosinmonophosphat), cGMP (cyclisches Guanozinmonophosphat) oder Ca²⁺ zu verstehen. Das intrazelluläre Signaling wird über Änderungen der Konzentration der second messengers gesteuert. Die G-Proteine bzw. deren Untereinheiten wechselwirken hierzu mit Proteinen wie der Adenylat-Zyklase, der Phospholipase C oder lonenkanälen. Die Änderung der Konzentration der "second messenger" wiederum bewirkt eine Aktivierung oder Deaktivierung von anderen Proteinen insbesondere von Kinasen und Phosphatasen. Das Signal endet schließlich in einer für den jeweiligen Zellverband typischen Antwort beispielsweise der Expression eines Proteins.

Die heterotrimeren G-Proteine liegen an der Innenseite der Plasmembran. Ein aktivierter Rezeptor nimmt Kontakt auf mit dem G-Protein-Heterotimer, welches daraufhin eine α-Untereinheit und den βγ-Komplex dissoziiert. Sowohl die aktivierte α-Untereinheit als auch der βγ-Komplex können intrazelluläre Effektorproteine beeinflussen. Die G-Protein-α-Untereinheit Familie kann in verschiedene Klassen eingeteilt werden. Bekannt sind beispielsweise die Gαs-, Gαi-, Gαq- und Gα12-Klasse. GPCRs werden entsprechend der aktivierten G-Proteine klassifiziert.

GPCRs der Gs-Klasse vermitteln über Aktivierung von Gαs die Stimulation der Adenylacyclase und erhöhen die intrazelluläre cAMP-Konzentration. GPCRs der Gi-Klasse bewirken über Aktivierung von Gαi eine Hemmung der Adenylatcyclase und erniedrigen das intrazelluläre cAMP.
GPCRs der Gq-Klasse wiederum erreichen über Aktivierung von Gαq eine Stimulation verschiedener PLCβ-Isoformen und führen über die Hydrolyse von membranständigem Phosphatidyl-inositol-4,5-biphosphat zu Diacylglycerol und Insositoltriphosphat (IP3). IP3 setzt aus intrazellulären Speichern Ca²⁺ frei. Die meisten GPCRs können nur mit einer G-Protein-β-Untereinheit Familie Kontakt aufnehmen, d.h., sie besitzen Selektivität für einen bestimmten Signaltransduktionsweg.

G-Proteine mit geänderter Rezeptorspezifität und unterschiedlicher Anbindung an einen Signaltransduktionsweg können durch Aneinanderfügen von Bestandteilen aus verschiedenen G-Proteinen zu Hybrid-G-Proteinen mittels der Methoden der Molekularbiologie und Biochemie konstruiert werden.
Hybrid-G-Proteine sind Fusionskonstrukte, die innerhalb eines Proteins Sequenzen verschiedener Gα-Untereinheiten vereinigen. So kann z. B. durch die Fusion der Rezeptorerkennungsregion von Gαi mit der Effektor-Aktivierungsregion von Gαq ein Gαq/i-Hybrid hergestellt werden, das Signale von Gi-gekoppelten Rezeptoren empfängt, aber den Gαq-PLCβ-Signaltransduktionsweg anschaltet. Ein derartiges Hybrid, bei welchem die C-terminalen 5-Aminosäuren von Gαq durch die entsprechende Gαi-Sequenz ersetzt worden ist (Gαiq5) wurde von Conklin et al. Nature 363, 274-276 (1993) erstmalig beschrieben.
Diese "Umkopplung" von Rezeptoren hat den Vorteil, daß der Assayendpunkt (Anstieg der intrazellulären Ca²⁺-Konzentration im Vergleich zur Hemmung der Adenylatzyklase) einfacher durch meßtechnische Verfahren zugänglich ist und im High-Troughput-Screening verwendet werden kann.

Sphingosin-1-phosphat (S1P) und Lysophosphatidsäure (LPA)) sind Lipidsignalmoleküle, welche aus Membranphosphlipiden gebildet werden. S1P und LPA sind hauptsächlich als intrazelluläre Signalmoleküle bekannt. Im Falle einiger GPCRs funktioniert S1P oder LPA als natürlicher Ligand. Als Ligand soll ein Molekül verstanden werden, welches reversibel an einen G-Protein gekoppelten Rezeptor bindet und über diese Bindung eine Wirkung auf den Rezeptor (Stabilisierung, Inaktivierung, Stimulierung) ausübt. Diese Wirkung betrifft im allgemeinen eine nachgeschaltete intrazelluläre Signalkaskade und kann über die Effekte aus der Signalkaskade nachgewiesen werden. Ein Ligand ist natürlich, wenn er von einem biologischen System hergestellt wird.

Die Nukleotid- und Aminosäuresequenz des GPR45like/GPR63 Rezeptors ist bekannt. (Genbank: NM_030784; TREMBL:Q9b2i6). In SEQ ID NO. 1 ist die Nukleotidsequenz und in SEQ ID NO. 2 die Aminosäuresequenz des GPR45like/GPR63 Rezeptors dargestellt. Nicht bekannt bislang ist ein natürlicher Ligand für den GPR45like/GPR63 Rezeptor. Damit können keine Agonisten oder Antagonisten für diese Rezeptorfunktion identifiziert werden. Agonisten oder Antagonisten sind in aller Regel definierte organische Moleküle mit präziser Struktur und einem nacharbeitbaren Herstellungsverfahren. Mittels solcher Verbindungen erst besteht die Möglichkeit, die Funktion dieses Rezeptors in unterschiedlichen Geweben, Entwicklungsstadien und unterschiedlichen äußeren Einflüssen zu untersuchen. Damit werden Hilfsmittel zur Verfügung gestellt, um die Rolle dieses Rezeptors in normalen und krankheitsmäßig veränderten Geweben untersuchen zu können. Aufgabe der vorliegenden Erfindung ist deshalb die Bereitstellung eines Verfahrens zur ldentifizierung einer Verbindung, welcher agonistisch oder antagonistisch auf den GPR45like/GPR63 Rezeptor wirkt.

Die Erfindung betrifft ein Verfahren zur Identifizierung einer Verbindung, welche die Aktivität des G Protein gekoppelten Rezeptors GPR45like/GPR63 modifiziert, wobei
a] biologisches Material oder eine Präparation aus biologischem Material bereitgestellt wird, welches den G Protein gekoppelten Rezeptor GPR45like/GPR63 sowie einen natürlichen Liganden dieses Rezeptors ausgewählt aus Sphingosin-1-phosphat oder Lysophosphatidsäure enthält,
b] eine chemische Verbindung bereitgestellt wird;
c] das biologische Material oder die Präparation aus biologischem Material gemäß a] mit der chemischen Verbindung gemäß b] in Kontakt gebracht wird
d] die Aktivität des G Protein gekoppelten Rezeptors GPR45like/GPR63 bestimmt wird.

Die Modifizierung der Aktivität des G Protein gekoppelten Rezeptors GPR45like/GPR63 besteht insbesondere in dessen Stabilisierung, Anschaltung, Abschaltung, Erhöhung oder Erniedrigung.

Das Inkontaktbringen der chemischen Verbindung an das biologische Material oder der Präparation daraus erfolgt unter Bedingungen, die eine Wechselwirkung ermöglichen. Hierbei ist insbesondere die Temperatur (Raumtemperatur bis ca. 37°C) sowie der pH-Wert (pH 6-8, bevorzugt pH ca. 7,0) von Bedeutung.

In einer bevorzugten Ausführungsform des Verfahrens wird ein G Protein gekoppelter Rezeptor GPR45like/GPR63 ausgewählt aus einer der folgenden Gruppen
a] ein Rezeptor bestehend aus der Aminosäuresequenz gemäß SEQ ID NO. 2,
b] ein Rezeptor, welchem hinsichtlich der Aminosäuresequenz von SEQ ID NO. 2 eine oder mehrere Aminosäuren fehlen,
c] ein Rezeptor, in welchem hinsichtlich der Aminosäuresequenz von SEQ ID NO. 2 eine oder mehrere Aminosäuren hinzugefügt sind.

Der G Protein gekoppelte Rezeptor GPR45like/GPR63 zur Durchführung des vorstehend beschriebenen Verfahrens kann bevorzugt kodiert werden aus einer der folgenden Gruppen:
a] einer Polynukleotidsequenz gemäß SEQ ID NO. 1,
b] einer Polynukleotidsequenz gemäß SEQ ID NO. 1, worin bedingt durch die Degeneriertheit des genetischen Codes eine oder mehrere der Nukleotidpositionen ein anderes Nukleotid enthalten.

Der G Protein gekoppelte Rezeptor GPR45like/GPR63 zur Durchführung des erfindungsgemäßen Verfahrens kann durch Expression einer exogenen DNA Sequenz in einem Prokaryonten oder Eukaryonten hergestellt werden. Grundsätzlich eignet sich zur Herstellung des Proteins jeder prokaryotische oder eukaryotische Plasmidvektor, Bakteriophagenvektor oder Hefeplasmidvektor. Beispiele für solche Vektoren sind pBR322, pUC18,19, pBlueScript, pcDNA3.1 und andere.

Der natürliche Ligand kann in dem biologischen Material oder der Präparation aus biologischem Material bereits vorhanden sein. Man kann einem solchen Liganden aber auch von außen zugeben. Hierzu sollte der Ligand in einer Menge, Konzentration und einem Reinheitsgrad vorliegen, daß dessen Bindung an den Rezeptor sowie die Auslösung des Rezeptorsignals bewirkt wird.

Die Zugabe des natürlichen Liganden erfolgt, nachdem das biologische Material oder eine Präparation des biologischen Materials, welche jeweils den G Protein gekoppelten Rezeptor GPR45like/GPR63 enthalten, in geeigneter Weise (Zubereitung/Menge) zur Verfügung steht.

Bei solch einem natürlichen Liganden handelt es sich um Sphingosin-1-phosphat oder Lysophosphatidsäure. Die natürlichen Liganden können zur Verwendung im erfindungsgemäßen Verfahren eine durch eine geeignete Detektionsmethode nachweisbare Markierung enthalten. Eine solche Markierung ist beispielsweise eine radioaktive Markierung oder eine fluorometrisch nachweisbare Markierung.

Das erfindungsgemäße Verfahren wird vorzugsweise so verwendet, daß die identifizierten Verbindungen agonistisch oder antagonistisch zum natürlichen Liganden wirken.

Die Beschreibung betrifft auch eine Verbindung, welche die Aktivität des G Protein gekoppelten Rezeptors GPR45like/GPR63 modifiziert, wobei die Verbindung durch ein Verfahren wie vorstehend beschrieben identifiziert wurde.

Eine solche Verbindung zeichnet sich dadurch aus, daß deren Masse bevorzugt zwischen 0,1 bis 50 kDa liegt, oder besonders bevorzugt zwischen 0,1 bis 5 kDa liegt oder ganz besonders bevorzugt zwischen 0,1 bis 3 kDa liegt.

Eine solche Verbindung, welche durch ein erfindungsgemäßes Verfahren identifiziert werden kann, kann ein Protein, eine Aminosäure, ein Polysaccharid, ein Zucker, ein Polynukleotid, ein Nukleotid, eine fettsäurehaltige Verbindung, ein Fett, eine Fettsäure, ein Fettsäurederivat oder eine aromatische Kohlenwasserstoffverbindung sein.

Die Beschreibung betrifft weiterhin ein Arzneimittel, welche eine Verbindung, die durch ein erfindungsgemäßes Verfahren identifiziert wurde, enthält, sowie weiterhin Formulierungshilfsstoffe für ein Arzneimittel und/oder weitere Zusatzstoffe. Dieses Arzneimittel eignet sich insbesondere zur Behandlung einer Herz-Kreislauferkrankung.

Eine Verbindung, welche durch ein erfindungsgemäßes Verfahren identifiziert, kann zur Herstellung eines Arzneimittels zur Behandlung einer Herz-Kreislauferkrankung verwendet werden.

Die Erfindung betrifft weiterhin die Verwendung von Sphingosin-1-phoshat oder Lysophosphatidsäure zur Herstellung eines Komplexes des Rezeptors GPR45like/GPR63 mit dieser Verbindung. Die Erfindung betrifft auch einen Komplex aus dem Rezeptor GPR45like/GPR63 sowie Sphingosin-1-phosphat und/oder Lysophosphatidsäure. Als Komplex soll hier ein Verbund aus einem oder mehreren Proteinen des Rezeptors in mindestens spezifischer Bindung mit Sphingosin-1-phosphat und/oder Lysophosphatidsäure sowie evtl. erforderlichen weiteren Lipiden, Membranbestandteilen oder Detergentien zur Stabilisierung des Rezeptors bzw. Rezeptor/Ligand-Komplexes verstanden werden. Die Bindung ist spezifisch, wenn die Bindekonstante kleiner oder gleich 100 µM beträgt.

Der G Protein gekoppelte Rezeptor GPR45like/GPR63 kann mittels eines rekombinanten Vektorkonstrukts kloniert und hergestellt werden.

Rekombinante Vektorkonstruktionen können unter Zuhilfenahme des einschlägigen Fachwissens, wie dargestellt beispielsweise in "F.M. Ausubel et al., Current Protocols in Molecular Biology, Wiley & Sons, New York (ISBN 0-471-50338-X)" oder om "J. Sambrook, E.F. Fritsch, T. Mamiatis, Molekular Cloning, second edition, Cold Spring Harbor Laboratory Press" (ISBN 0-87969-309-6), hergestellt werden. Dabei wird ein Polynukleotid kodierend für eine Aminosäuresequenz gemäß einer der beschriebenen Sequenzinformationen (SEQ ID NO. 1) oder eine Polynukleotidsequenz gemäß einer der beschriebenen Sequenzinformationen (SEQ ID NO. 2) in einen Basisvektor eingebaut. Als Basisvektor soll ein Vektor verstanden werden, in welchen eine Polynukleotidsequenz eines Polynukleotids mittels der Methoden der Molekularbiologie eingebaut und in einem Mikroorganismus beispielsweise einem Bakterium, Pilz oder der Zelle einer Zellkultur kloniert werden kann. Der Basisvektor kann beispielsweise aus einem Plasmid mit einem Antibiotikumresistenzmarker, einem "origin of replication" geeignet zur Vermehrung des Plasmids in Bakterien oder Zellkulturen, sowie einem Promotor geeignet zur Expression eines Proteins bestehen. Der Basisvektor kann beispielsweise auch aus einem Phagenvektor, einem Phagemidvektor, einem Phasmidvektor, einem Cosmidvektor, einem Virusvektor, einem YAC-Vektor oder anderen Vektortyp bestehen. Beispiele für Basisvektoren sind pUC 18, pUC19, pBLuescript, pKS, pSK und andere. Der Einbau des einzubauenden Polynukleotids erfolgt über geeignete Restriktionsschnittstellen mittels der entsprechenden Restriktionsenzyme, welche kommerziell von Firmen wie BioLabs, Roche Diagnostics, Stratagene und anderen angeboten werden. Solche Restriktionsschnittstellen können beispielsweise die Erkennungsstellen der Restriktionsenzyme BamHl, EcoRl, Sall, EcoRV und andere sein.

Die rekombinante Vektorkonstruktion besteht in einer bevorzugten Ausführungsform aus einem in Eukaryonten und/oder Prokaryonten verwendbaren Expressionsvektor. Ein Expressionsvektor enthält einen Promotor, der funktionell mit einer Polynukleotidsequenz verbunden werden kann, so daß ein von dieser Polynukleotidsequenz kodiertes Protein in einem biologischen Organismus beispielsweise einem Bakterium, Pilz oder der Zelle einer eukaryotischen Zelllinie synthetisiert wird. Der Promotor kann induzierbar sein beispielsweise mittels Tryptophan oder er kann konstitutiv aktiv sein. Beispiele für Expressionsvektoren sind pUC18, pUC19, pBluescript, pcDNA3.1 oder andere.

Biologisches Material ist jedes Material, das genetische Informationen enthält und sich selbst reproduzieren oder in einem biologischen System reproduziert werden kann. Biologisches Material sind beispielsweise Zellen aus menschlichen oder tierischen Geweben oder Organen wie insbesondere Gehirn, Fettgewebe, Lunge, Herz, Leber, Niere, Milz, Muskel und andere. Biologisches Material sind beispielsweise auch Bakterien oder Pilze wie beispielsweise Escherichia coli oder Saccharomyces cerevisiae. Weiterhin umfaßt biologisches Material Zellen aus Zellkulturen.
Die Gewinnung von biologischem Material kann im Falle von Zellen aus tierischen oder menschlichen Geweben durch Biopsie, operative Entnahme, Entnahme mittels Spritzen oder Kathetern oder vergleichbaren Techniken erfolgen. Die so entnommenen Zellen können tiefgefroren, aufgearbeitet oder in Zellkultur genommen werden. Bakterien und Hefezellen werden mittels gebräuchlicher Techniken der Mikrobiologie vermehrt und aufgearbeitet.

Entsprechende Anleitungen hierfür findet der Fachmann in "Current Protocols in Molecular Biology; ed.: F. M. Ansubel et al., laufend erneuert, Ausgabe 2001, Verlag John Wiley & Sons".
Biologisches Material kann auch aus den Zellen einer tierischen Zellkultur bestehen. Solche Zellen sind beispielsweise Mauszellen, Rattenzellen oder Hamsterzellen. Die Zellkulturzellen können primäre Zelltypen oder etablierte Zelllinien sein. Beispiele für etablierte Zelllinien sind Maus 3T3-Zellen, CHO-Zellen oder Hela-Zellen. Haltung, Anzucht und Vermehrung von Zelllinien ist in Standardlehrbüchem beschrieben, wie beispielsweise in "Basic Cell Culture; ed.: J. M. Davis IRL Press, Oxford (1996).

Eine Präparation eines biologischen Materials wird beispielsweise durch Aufschluß des biologischen Materials und sich daran anschließende Reinigungsschritte hergestellt. Methoden zum Aufschluß des biologischen Materials können insbesondere wiederholtes Einfrieren und Auftauen, die Behandlung mit Ultraschall, die Verwendung einer French-Press, die Zugabe von Detergentien und Enzymen oder vergleichbares sein. Sich anschließende Reinigungsschritte bestehen beispielsweise aus differentieller Zentrifugation, der Fällung mit Ammonsulfat oder organischen Lösungsmitteln, der Anwendung von chromatographischen Techniken und anderen. Chromatographische Techniken sind beispielsweise die Polyacrylamidgelektrophorese, Hochdruckflüssigkeitschromatographie, lonenaustauschchromatograhie, Affinitätschromatographie, Gaschromatograhie, Massenspektrometrie und anderes. Hierfür und insbesondere auch für detaillierte Anweisungen zur Reindarstellung von Proteinen stehen dem Fachmann detaillierte Anweisungen in Lehrbüchern zur Verfügung wie insbesondere in "Current Protocols in Protein Science, ed.: J.E. Coligan et al., laufend erneuert, Ausgabe 2001, Verlag John Wiley & Sons.

Das Inkontaktbringen des biologischen Materials oder der Präparation aus biologischem Material mit einer chemischen Verbindung kann in gewöhnlichen Laborgefäßen wie beispielsweise Eppendorf-Gefäßen, Zentrifugen-Röhrchen oder Glaskolben erfolgen. Das zugrundeliegende wäßrige Medium enthält beispielsweise Puffersubstanzen, Nährmediumbestandteile, einwertige oder zweiwertige Ionen wie Na⁺, K⁺, Ca²⁺, ,Cl⁻, SO₄²⁻, PO₃²⁻ oder andere, weiterhin Proteine, Glycerin oder anderes. Für das Inkontaktbringen können bestimmte konstante Bedingungen wie insbesondere die Temperatur, der pH-Wert, die lonenbedingungen, die Konzentration eines Proteins, des Volumens oder andere Faktoren vorteilhaft sein. Dies wird erreicht, in dem beispielsweise das Inkontaktbringen in konstant temperierten Inkubationsvorrichtungen, in Gegenwart eines Puffers oder mit den zuvor genau eingewogenen Mengen der Ionen oder Proteine durchgeführt wird. Das wäßrige Lösungsmittel kann insbesondere auch einen bestimmten Anteil eines organischen Lösungsmittels wie Dimethylsulfoxid, Methanol oder Ethanol enthalten. Der Gehalt eines solchen Lösungsmittels beträgt aber vorzugsweise nicht mehr als 10 Vol. % des Ansatzes.

Die Bereitstellung einer chemischen Verbindung erfolgt beispielsweise durch chemische Synthese. Dem Fachmann sind die Standardsynthesemethoden geläufig. Die chemische Verbindung kann Teil einer Sammlung chemischer Verbindungen sein, wie sie durch Lagerung und Katalogisierung der chemischen Verbindungen aus abgeschlossenen Syntheseprogrammen entstehen (sogenannte "chemical libraries"). Die Verbindung kann in anderen Fällen von einem Mikroorganismus insbesondere einem Bakterium, aber auch von einem Pilz oder einer Pflanze gebildet worden sein (Naturstoff).

Geeignete pharmazeutische Verbindungen können als Arzneimittel in oraler, peroraler, topischer, parenteraler oder rektaler Form verabreicht werden. Die am besten geeignete Verabreichungsweise ist in jedem Einzelfall von der Art und Schwere des zu behandelnden Zustands und von der Art der jeweils verwendeten Verbindung abhängig. Eine geeignete Wirkstoffkonzentration beträgt ca. 1 % bis 35 %, vorzugsweise ca. 3 % bis 15 %.

### Beispiele

### Klonierung des humanen GPR45like/GPR63 Rezeptors

Die Sequenz des humanen GPR45 like Rezeptors ist unter den Nummern AF 31765 und AB 030566 bei Genbank und EMBL öffentlich zugänglich. Das humane Gen enthält keine Introns. Der Rezeptor wurde deshalb ausgehend von humaner genomischer DNA mittels einer Polymerasekettenverlängerungsreaktion (Polymerase Chain Reaktion; PCR) vervielfältigt. Die PCR Reaktionsbedingungen waren die folgenden:
Zuerst 10 Min. Inkubation bei 94°C, dann 35 Zyklen pro Zyklus jeweils 1 Min. Inkubation bei 94°C, dann 1 Min. Inkubation bei 60°C und schließlich 2 Min. Inkubation bei 72°C. Die Reaktion wurde in diesem Fall mittels des GC-melt Kit der Firma Clontech ausgeführt. Die verwendeten Primer gemäß SEQ ID Nr. 3 und 4 wurden so konstruiert, daß sie eine Hindlll-Schnittstelle (SEQ ID Nr. 3) und eine EcoRl-Schnittstelle (SEQ ID Nr. 4) enthielten. Das durch zuvor beschriebene PCR-Reaktion gebildete PCR-Fragment mit einer Länge von 1260 Basenpaaren wurde mit Hilfe der Hindlll- und EcoRI-Schnittstellen in den eukaryotischen Expressionsvektor pCDNA 3.1 (+) eingebaut. Dieser Vektor ist kommerziell erhältlich beispielsweise bei der Firma Invitrogen, Carlsbad, Kalifornien.

### Extraktion von RNA; PCR mittels reverser Transkriptase (RT-PCR)

Aus verschiedenen Zelllinien wurde RNA isoliert. Im vorliegenden Fall wurde dazu TRIzol Reagenz der Firma Gibco BRC verwendet.
Die RNA Isolierung erfolgte aus folgenden Zelllinien: HUVECS (human unbelical vein endothelial cells). HPAEC (human pulmonary artery endothelial cells), Hek 293 (human embryonic kidney cells), HCASMC (human coronary artery smooth muscle cells), HCAEC (human coronary artery endothelial cells), HMVEC-L (human microvascular endothelial cells of the lung), HPASMC (human pulmonary artery smooth muscle cells), HAOSMC (human aortic smooth muscle cells).

Die Zellen wurden kurz vor Erreichen der Konfluenz in einer Gewebekulturflasche geerntet. Aus diesen Zellen wurde RNA nach den Angaben des Herstellers für TRI zol Reagenz isoliert. Die RNA wurde auf Abwesenheit von genomischer DNA getestet. Mit ca. 5 µg dieser RNA wurde eine reverse Transkriptase Reaktion mittels der MMLV (Moloney Murine Leukemia Virus) reversen Transkriptase und des RT-PCR Kits der Firma Stratagene durchgeführt. Die RT-PCR wurde in einem Volumen von 50 µl ausgeführt, wobei bei der Durchführung der Reaktion zuerst 5 Min. bei 65°C, dann 15 Min. bei Raumtemperatur, dann 1 Stunde bei 37°C, dann 5 Min. bei 90°C inkubiert und schließlich in Eis abgekühlt wurde.

Für die nachfolgenden PCR Reaktionen wurden die cDNA Präparationen aus diesen reversen Transkriptionen verwendet. Soweit verfügbar wurde kommerziell erhältliche cDNA eingesetzt.

Für eine PCR verwendete man ca. 5 µg cDNA. Die Reaktion selbst wird mit einem Amplitaq Gold Polymerase Kit der Firma Perkin Elmer durchgeführt. Die Reaktionsbedingungen für die Zyklen sind die folgenden:
12 Min. Inkubation bei 95°C, dann 35 Zyklen für jeden Zyklus jeweils zuerst 1 Min. Inkubation bei 94°C, dann 1 Min. Inkubation bei 72°C, dann nach Durchlauf der 35 Zyklen 10 Min. Inkubation bei 72 °C und schließlich Abkühlen auf Eis.

Als Primer wurden hierfür die beiden folgenden DNA-Sequenzen verwendet:
5'-CCC ACT GGT TTG AGT TCC TTG ACC-3' (SEQ ID Nr. 5; "vorwärts"),
5'-GGT AGC CTG GAT TGG TTG TGT ACC-3' (SEQ ID Nr. 6; "rückwärts")

Es entstand ein 561 Basenpaare langes Produkt.

### Quantitative RT-PCR in Echtzeit mittels Taqman

Die quantitative PCR Analyse wurde mittels Fluoreszenz Resonanz Energie Transfer (FRET) durchgeführt. Dieses System ist dem Fachmann auch als Taqman-PCR geläufig. Für die Durchführung der Taqman Reaktion wurde ein kommerziell erhältlicher Kit von Perkin Elmer verwendet. Die Taqman-Probe besteht aus einem einzelsträngigen Oligonukleotid, welches mit 2 verschiedenen Fluorophoren markiert ist. Das Fluorophor am 3'-Ende (Acceptor) wirkt als "quencher" (d.h. mit abschwächender Wirkung) des Fluorophors am 5'-Ende (Donor). Die Taq DNA Polymerase setzt durch ihre 5'-Exonuclease-Aktivität während der Kettenverlängerungsreaktion das Fluorophor des 5'-Endes frei. Da die Emission des Fluorophors nun nicht mehr gequencht wird, kann sie über ein Fluorimeter gemessen werden. Die Menge an festgestellter Fluoreszenz ist direkt proportional zur Menge des PCR-Produkts, welches während der Amplifikation akkumuliert. Es ist darauf zu achten, daß die Schmelztemperatur des Taqman-Oligos höher ist als diejenige des Primers für die Amplifizierung durch die Taq-Polymerase.

### Als Fluorophore wurden im vorliegenden Fall

FAM (6-Carboxyflusrescein) als Donor für das 5'-Ende und TAMRA (5-Carboxytetramethylrhodamin) als Acceptor/quencher für das 3'-Ende verwendet.

Zur Herstellung der cDNA verwendete man kommerziell erhältliche RNA beispielsweise der Firma Clontech, Heidelberg.

Man mischte 5 µg von Gesamt-RNA mit 2,5 µl (5 mg/µl) von Hexamerprimern mit zufälliger Sequenz. Solche Hexamerprimer werden von verschiedenen Firmen angeboten. Im vorliegenden Fall stammten sie von der Firma Invitrogen (Life Technologies), Karlsruhe.
Gesamt-RNA und Hexamerprimer wurden zuerst 10 Min. für 70°C erhitzt und auf Eis abgekühlt. Dann wurden 4 µl 5-fach Puffer (First stand puffer), 2 µl von 0,1 nM DTT (Dithiothreitol), 1 µl von 10 mM dNTP und 1 µl Wasser zugegeben, vorsichtig vermischt und für 2 Minuten bei 37°C inkubiert. Daraufhin ergänzte man mit 5 µl reverser Transkriptase und inkubierte für 60 Min. bei 37°C. Die Reaktion wurde durch Zugabe von 1 µl 2,5 mM EDTA und Erhitzen für 10 Min. auf 65°C unterbrochen.
Die so hergestellten cDNA-Proben dienten als Matrizen für die anschließende quantitative PCR Reaktion.

Von jeder revers transkritierten cDNA Matrize wurden jeweils 50 ng/µl, 25 ng/µl, 10 ng/µl, 5 ng/µl, 2,5 ng/µl, 1,25 ng/µl und 0,625 ng/µl Endkonzentration untersucht.
Die Reaktion erfolgte in einem Gesamtvolumen von 50 µl. Der Reaktionsansatz enthielt dNTP und Puffer in den üblichen Konzentrationen sowie Taq Polymerase.

Die Endkonzentration der Primer betrug jeweils 900 mM.

Es wurde ein Abgleich vorgenommen mit Primern für das humane Beta Aktin Gen in menschlicher cDNA aus Gehirn.

Die Bestimmungen wurden jeweils zweifach in verschiedenen Ansätzen durchgeführt. Die Standardwerte für das humane Aktin Gen wurden als interne Kontrollen zur Normalisierung der Proben bei der Bestimmung der zu ermittelnden Expression des GPR45like/GPR63 Gens in den verschiedenen RNAs verwendet. Die Expression wurde in Relation zu einem vorher festgelegten Referenzgewebe ausgedrückt. Für das zentrale Nervensystem wurde das Kleinhirn und für das periphere Gewebe wurde das Gehirn jeweils als Bezugswert festgelegt.

Die verwendeten Primer für die Amplifizierungsreaktion der ersten Bestimmung hatten folgende Nukleotidsequenz
5'- CTC AAC ACC CTT CGC CAC A-3' (SEQ ID Nr. 7)
5'-GGCC TGG CTG AGG CAT ATAC-3' (SEQ ID Nr. 8).

Die Taqman Oligo Probe hierfür hatte die Nukleotidsequenz:
5'-TGC CTT GAG GAT CCA TAG CTA CCC TGA A-3' (SEQ ID Nr. 9).

Die Primer der Amplifizierung der zweiten Bestimmung hatten die folgende Nukleotidsequenz:
5'-TGCC TGG ACA TGA TGC CTA A-3' (SEQ ID Nr. 10)
5'-TCC GTC GCT TTG TGT GAC C-3' (SEQ ID Nr. 11).

Die entsprechende Taqman Probe hierfür hatte die Nukleotidsequenz:
5'-TCC TTC AAC TTT TTG CCG CAG CTC C-3' (SEQ ID Nr. 12).

### Northern Blot

Northern Blots mit RNA aus unterschiedlichen menschlichen Geweben wurden von Clontech, Palo Alto gekauft. Solche Northern Blots sind auch von anderen Anbietern in derselben Qualität kommerziell erhältlich. Die Nukleotidsequenz des GPR45like/GPR63 Rezeptors wurde mittels EcoRI und HindIII aus einem pcDNA 3.1 abgeleiteten Expressionsplasmid ausgeschnitten, über ein Agarosegel aufgetrennt, die 1260 Basenpaar lange Sequenz wurde isoliert und anschließend mit 32^{P}-dCTP radioaktiv markiert. Die Northern Blots mit der RNA aus unterschiedlichen Geweben wurden unter stringenten Bedingungen hybridisiert. Die Radioaktivität wurde anschließend mittels eines Films nachgewiesen. Zum internen Abgleich wurden dieselben Northern Blots so lange gewaschen, bis keine der zuvor hybridisierten DNA Moleküle mit der kodierenden Sequenz für den GPR45like/GPR63 Rezeptor mehr nachzuweisen waren. Diese gewaschenen Northern Blots wurden erneut hybridisiert, wobei eine beta-Actin cDNA Probe verwendet wurde.

### Nachweis der GPR45like/GPR63 Rezeptor Aktivität in transfizierten Zellkulturen

CHO-K1 Zellen wurden in basalem Iscove-Medium mit weiteren Zusätzen kultiviert. Iscove-Medium ist kommerziell erhältlich z.B. von Biochrom. Die Zusammensetzung wurde erstmals beschrieben in "Iscove, N.N., Melchers, F., Journal of Experimental Medicine 147, 923-933 (1978). Als weitere Zusätze zum Iscove-Medium wurden 10 % fötales Rinderserum, 10 000/U/ml-10 000 µg/ml Penicillin-Streptomycin, Gentamycin, und 2 mM L-Glutamin verwendet. Die Zellen wurden bei 37°C und einer 5 % CO₂-Atmosphäre inkubiert.
Für die transiente Transfektion wurden ca. 2 x 10⁵ CHO-K1 Zellen in 35 mm Schalen ausgebracht. Nach Weiterinkubation für etwa 24 Std. und bei einer Konfluenz der Zellen von ca. 50-80 % wurden die Zellen mit 1 µg des Plasmid-DNA Konstrukts unter Zuhilfenahme von Lipofectamin (Gibco) transient transfiziert.

### FLIPR-Assay (Fluorometric Imaging Plate Reader Assay)

Nach ca. 16 bis 18 Stunden nach der Transfektion wurden die CHO-Zellen in einer Menge von ca. 80 000 Zellen pro Vertiefung in 96-well Platten gegeben und für etwa 18-24 weitere Stunden inkubiert. Zu den Zellen wurden 95 µl HBSS (Hank's buffered Saline Solution) mit 20 mM HEPES, 2,5 mM Probenecid (4-[(dipropylamino)sulfonyl]benzolsäure) 4 µM dyeFluo4 gegeben.

Die Zellen wurden für 1 Stunde in 5 % CO₂ inkubiert und dreimal mit PBS (Phosphate buffered Saline), welche 1 mM MgCl₂, 1 mM MEDTA, 0,4 mg/ml FAF-BSA (Fatty acid free bovine serum albumin) und 2,5 mM Probenecid enthielt, gewaschen.

Nach dem letzten Waschschritt wurden 100 µl in jeder Vertiefung auf den Zellen belassen. Die zu testenden Lipide standen als 2 mM Stammlösung in DMSO (Dimethylsulfoxid) zur Verfügung. Auf einer 96 Well Mikrotiterplatte wurde eine 60 µM Lösung bereitgehalten. Die Verdünnung aus der Stammlösung erfolgte in PBS. Von dieser 6 µM Lösung überführte man jeweils 50 µl in jede Vertiefung derjenigen Mikrofilterplatte, welche 100 µl PBS sowie die Zellen enthielt. Man erhielt so eine 20 µM Endkonzentration der zu testenden Lipide.

Die Fluoreszenz wurde vom Gerät (FLIPR, Molecular Devices) 1 Min. lang in 1 Sek. dauernden Intervalle und weitere 2 Min. lang in 3 Stunden Intervallen gemessen.

### Zellvermehrungs-Assay

CHO Zellen wurden in einer Menge von 8 x 10⁴ Zellen in 35 mm Platten gegeben. Nach 32 Stunden transfizierte man die Zellen mit 1 µg des Plasmidkonstrukts mit Hilfe von Lipofectamine (Gibco). Nach weiteren 13 Stunden (Zeitpunkt O) wurden die Zellen mit PBS gewaschen und 48 Stunden (Zeitpunkt 48) in Iscove Medium, welches 10 % dialysiertes fötales Kälberserum enthielt, inkubiert und zwar in Gegenwart oder Abwesenheit von 1 µM S1P (Sphingosin-1-Phosphat).

Die Anzahl der Zellen beim Zeitpunkt 0 und Zeitpunkt 48 wurde durch Auszählen bestimmt. Hierzu wurden die Zellen zuerst mit Trypsin behandelt und anschließend in 1 ml Iscove suspendiert. Eine Menge von 100 ml dieser Zellen wurde in 10 ml Puffer verdünnt und in einem Zellcounter z.B. im Casy Cellcounter TT (Cell Counter und Analysesystem, Schärfe, Reutlingen) ausgezählt.
Spezifität der Expression des GPR45like/GPR63 Rezeptors in verschiedenen humanen Geweben

Die Expression wurde mittels der RT-PCR untersucht. Spezifische Transkripte des GPR45like/GPR63 Rezeptors konnte in Aorta, Herz, dem linken Ventrikel, fötalem Herzen, Gehirn und der Niere nachgewiesen werden. In Geweben aus dem linken Atrium war nur eine schwache Bande zu gewinnen. Im Lungengewebe war kein Signal nachweisbar.

### Expression des GPR45like/GPR63 Rezeptors in verschiedenen humanen Zelllinien

Untersucht wurden HUVECS (human umbilical vein endothelial cells), HCAEC (human coronary artery endothelial cells), MHVEC-L (human microvascular endothelial cells from lung), HPAEC (human coronary artery smooth muscle cells), HPASMC (human pulmonary artery smooth muscle cells), HAOSMC (human aortic smooth muscle cells). Trankripte des GPR45like/GPR63 Rezeptors waren in allen Zelllinien nachzuweisen.

### GPR45like/GPR63 Rezeptor Expression in peripherem humanen Gewebe

Die Expression des Rezeptors wurde mittels der Taq Man RT-PCR Analyse semiquantitativ in Relation zur Expression im Gehirn bestimmt.

Ein interner Abgleich erfolgte in Bezug auf die β-Aktin RNA. Jeder Meßwert wurde zweifach bestimmt. Die folgende Tabelle zeigt die RNA Expression als vielfaches der Expression zum Gehirn.

**Tabelle:**

| Expression des GPR45like/GPR63 Rezeptors in verschiedenen Geweben in Relation zum Gehirn | |
|---|---|
| | Expression relativ zu Gehirn |
| Gehirn | 1 |
| Herz | 0,1 |
| Niere | 0,2 |
| Leber | 0,01 |
| Lunge | 0,05 |
| Milz | 0,2 |
| Thymus | 0,6 |
| Skelett-Muskel | 0,05 |
| Pankreas | 0,15 |
| Dünndarm | 0,8 |
| Magen | 0,7 |

Relative starke Expression zeigte sich im Thymus, Dünndarm und Magen während in Leber, Lunge und Skelett-Muskel fast keine Expression nachweisbar war.

GPR45like/GPR63 Rezeptor Expression in verschiedenen Teilen des Gehirn.

Die Expression des Rezeptors wurde mittels der Taq Man RT-PCR semiquantitativ in Relation zur Expression im Kleinhirn bestimmt. Der interne Abgleich wurde in Bezug auf die β-Aktin RNA vorgenommen. Jeder Meßwert wurde zweifach bestimmt. Die folgende Tabelle zeigt die RNA zum Kleinhirn

**Tabelle:**

| Expression des GPR45like/GPR63 Rezeptors in verschiedenen Teilen des Gehirn in Relation zum Kleinhirn | |
|---|---|
| | Expression relativ zum Kleinhirn |
| Kleinhirn | 1 |
| Gesamthim | 1,2 |
| Corpus callosum | 0,2 |
| Candatum | 1,5 |
| Thalamus | 2,1 |
| Amygdala | 1,2 |

Die vergleichsweise stärkste Expression beobachtete man im Thalamus.

### Stimulierung der intrazellulären Ca²⁺-Freisetzung durch den GPR45like/GPR63 Rezeptor

Zu CHO-Zellen, welche den GPR45like/GPR63 Rezeptor und die G Protein α Untereinheit delta 6qi4mgr (= i49i4) exprimierten, wurden 209 verschiedene bioaktive Lipide einer Substanzbibliothek jeweils in einer Endkonzentration von 1 µM gegeben. Getested wurden ca. 30 Verbindungen. Lediglich S1 P (Sphingosin-1-Phosphat) und DHS1P (Dihydrosphingosin-1-Phosphat) führten zu einem meßbarem Ca²⁺-Einstrom. S1P und DHS1P sind natürlicherweise vorkommende lipidähnliche Substanzen, die auch als natürliche Liganden anderer GPCRs in Erscheinung treten. Die *α* Untereinheit delta 6qi4mgr (= i4qi4) hat eine breite Spezifität bezüglich unterschiedlichen GPCRs.

Induktion der Zellvermehrung durch GPR45like/GPR63 Rezeptoren.

Eine Menge von 8 x 10⁴ CHO Zellen wurde auf 35 mm Platten ausgebracht. Die Zellen wurden nach 32 Stunden mit 1 µg DNA der Vektorkonstruktion transfiziert. Nach 13 Stunden wurden die Zellen einmal mit PBS gewaschen und für weitere 48 Stunden in Iscove Medium, welches 10 % dialysiertes fötales Kälberserum enthielt, in Gegenwart oder Abwesenheit von 1 µM S1P. Es konnte gezeigt werden, daß Versuchsansätze, welche S1P enthielten, im Durchschnitt von 2 unabhängig voneinander gemachten Versuchen ca. 20 % mehr Zellen enthielten.

## Patentansprüche

1. Verfahren zur Identifizierung einer Verbindung, welche die Aktivität des G Protein gekoppelten Rezeptors GPR451 ike/GPR63 modifiziert, wobei
a] biologisches Material oder eine Präparation aus biologischem Material bereitgestellt wird, welches jeweils den G Protein gekoppelten Rezeptor GPR451ike/GPR63 sowie einen natürlichen Liganden dieses Rezeptors ausgewählt aus Sphingosin-1-phosphat oder Lysophosphatidsäure enthält;
b] eine chemische Verbindung bereitgestellt wird ;
c] das biologische Material oder die Präparation aus biologischem Material gemäss a] mit der chemischen Verbindung gemäss b] in Kontakt gebracht wird ;
d] die Aktivität des G Protein gekoppelten Rezeptors GPR451ike/GPR63 bestimmt wird.

2. Verfahren nach Anspruch 1, **dadurch gekennzeichnet, dass** der Rezeptor aus einer der folgenden Gruppen ausgewählt wird
a] ein Rezeptor bestehend aus der Aminosäuresequenz gemäss SEQ ID Nr. 2;
b] ein Rezeptor, welchem hinsichtlich der Aminosäuresequenz von SEQ ID Nr. 2 ein oder mehrere Aminosäuren fehlen;
c] ein Rezeptor, in welchem hinsichtlich der Aminosäuresequenz von SEQ ID Nr. 2 ein oder mehrere Aminosäuren hinzugefügt sind.

3. Verfahren nach Anspruch 1, **dadurch gekennzeichnet, dass** der Rezeptor durch eine Polynukleotidsequenz aus einer der folgenden Gruppen kodiert wird,
a] eine Polynukleotidsequenz gemäss SEQ ID Nr. 1;
b] eine Polynukleotidsequenz gemäss SEQ ID Nr. 1, worin bedingt durch die Degeneriertheit des genetischen Codes eine oder mehrere der Nukleotidpositionen ein anderes Nukleotid enthalten.

4. Verfahren nach einem oder mehreren der Ansprüche 1 bis 3, **dadurch gekennzeichnet, dass** der Rezeptor durch prokaryotische oder eukaryotische Expression einer exogenen DNA Sequenz enthaltend eine Polynukleotidsequenz gemäss SEQ ID Nr. 1 hergestellt wird.

5. Verfahren nach Anspruch 4, **dadurch gekennzeichnet, dass** der natürliche Ligand von außen zugegeben wird, nachdem biologisches Material oder eine Präparation aus biologischem Material, welches jeweils den G-Protein gekoppelten Rezeptor GPR451 ike/GPR63 enthält, bereitgestellt wurde.

6. Verfahren nach einem oder mehreren der Ansprüche 1 bis 5, wobei der natürliche Ligand eine radioaktive Markierung oder eine fluorometrisch nachweisbare Markierung enthält.

7. Verfahren nach einem oder mehreren der Ansprüche 1 bis 6, wobei die Verbindung, welche die Aktivität des G-Protein gekoppelten Rezeptors GPR451ike/GPR63 modifiziert, agonistisch oder antagonistisch zum natürlichen Liganden wirkt.

8. Verwendung einer Verbindung ausgewählt aus Sphingosin-1-phosphat und/oder Lysophosphatidsäure zur Herstellung eines Komplexes aus dem Rezeptor GPR451ike/GPR63 und der Verbindung Sphingosin-1-phosphat und/oder Lysophosphatidsäure.

9. Protein enthaltend einen Komplex aus dem Rezeptor GPR451ike/GPR63 sowie Sphingosin-1-phosphat und/oder Lysophosphatidsäure.

## Claims

1. A method for identifying a compound which modifies the activity of the G protein-coupled receptor GPR451ike/GPR63, where
a] biological material or a preparation of biological material which contains the G protein-coupled receptor GPR451ike/GPR63 and a natural ligand of this receptor selected from sphingosine 1-phosphate or lysophosphatidic acid is provided,
b] a chemical compound is provided;
c] the biological material or the preparation of biological material from a] is brought into contact with the chemical compound from b];
d] the activity of the G protein-coupled receptor GPR451ike/GPR63 is determined.

2. The method as claimed in claim 1, wherein the receptor is selected from one of the following groups
a] a receptor consisting of the amino acid sequence as shown in SEQ ID NO. 2.
b] a receptor which is lacking one or more amino acids in relation to the amino acid sequence of SEQ ID NO. 2,
c] a receptor in which one or more amino acids have been added in relation to the amino acid sequence of SEQ ID NO. 2.

3. The method as claimed in claim 1, wherein the receptor is encoded by a polynucleotide sequence from one of the following groups
a] a polynucleotide sequence as shown in SEQ ID NO. 1;
b] a polynucleotide sequence as shown in SEQ ID NO. 1 in which, owing to the degeneracy of the genetic code, one or more of the nucleotide positions contain a different nucleotide.

4. The method as claimed in one or more of claims 1 to 3, wherein the receptor is produced by prokaryotic or eukaryotic expression of an exogenous DNA sequence comprising a polynucleotide sequence as shown in SEQ ID No. 1.

5. The method as claimed in claim 4, wherein the natural ligand is added from outside after biological material or a preparation of biological material which in each case comprises the G protein-coupled receptor GPR45like/GPR63 has been provided.

6. The method as claimed in one or more of claims 1 to 5, where the natural ligand comprises a radioactive label or a label detectable by fluorimetry.

7. The method as claimed in one or more of claims 1 to 6, where the compound which modifies the activity of the G protein-coupled receptor GPR451ike/GPR63 acts as agonist or antagonist of the natural ligand.

8. The use of a compound selected from sphingosine 1-phosphate and/or lysophosphatidic acid for producing a complex of the receptor GPR45like/GPR63 and the compound sphingosine 1-phosphate and/or lysophosphatidic acid.

9. A protein comprising a complex of the receptor GPR45like/GPR63 and sphingosine 1-phosphate and/or lysophosphatidic acid.

## Revendications

1. Procédé pour identifier un composé qui modifie l'activité du récepteur de type GPR45/GPR63 couplé à la protéine G, dans lequel
a] de la matière biologique ou une préparation d'une matière biologique est préparée, qui contient à chaque fois le récepteur de type GPR45/GPR63 couplé à la protéine G ainsi qu'un ligand naturel de ce récepteur choisi parmi le sphingosine-1-phosphate ou l'acide lysophosphatidique ;
b] un composé chimique est préparé ;
c] la matière biologique ou la préparation d'une matière biologique selon a] est mise en contact avec le composé chimique selon b] ;
d] l'activité du récepteur de type GPR45/GPR63 couplé à la protéine G est déterminée.

2. Procédé selon la revendication 1, **caractérisé en ce que** le récepteur est choisi dans un des groupes suivants
a] un récepteur constitué par la séquence d'acides aminés selon la SEQ ID Nr. 2 ;
b] un récepteur dans lequel il manque un ou plusieurs acides aminés par rapport à la séquence d'acides aminés de la SEQ ID Nr. 2 ;
c] un récepteur dans lequel sont ajoutés un ou plusieurs acides aminés par rapport à la séquence d'acides aminés de la SEQ ID Nr. 2.

3. Procédé selon la revendication 1, **caractérisé en ce que** le récepteur est codé par une séquence de polynucléotides d'un des groupes suivants,
a] une séquence de polynucléotides selon la SEQ ID Nr. 1 ;
b] une séquence de polynucléotides selon la SEQ ID Nr. 1 ;
dans laquelle, en raison de la dégénérescence du code génétique, une ou plusieurs des positions de nucléotides contiennent un autre nucléotide.

4. Procédé selon l'une ou plusieurs des revendications 1 à 3, **caractérisé**
**en ce que** le récepteur est préparé par une expression procaryote ou eucaryote d'une séquence d'ADN exogène contenant une séquence de polynucléotides selon la SEQ ID Nr. 1.

5. Procédé selon la revendication 4, **caractérisé**
**en ce que** le ligand naturel est ajouté à partir de l'extérieur, après que de la matière biologique ou une préparation d'une matière biologique, qui contient à chaque fois le récepteur de type GPR45/GPR63 couplé à la protéine G, a été préparée.

6. Procédé selon l'une ou plusieurs des revendications 1 à 5, où le ligand naturel contient un marquage radioactif ou un marquage pouvant être détecté par fluorimétrie.

7. Procédé selon l'une ou plusieurs des revendications 1 à 6, le composé, qui modifie l'activité du récepteur de type GPR45/GPR63 couplé à la protéine G agissant de manière agoniste ou antagoniste par rapport aux ligands naturels.

8. Utilisation d'un composé choisi parmi le sphingosine-1-phosphate et/ou l'acide lysophosphatidique pour la préparation d'un complexe du récepteur de type GPR45/GPR63 et du composé sphingosine-1-phosphate et/ou acide lysophosphatidique.

9. Protéine contenant un complexe du récepteur de type GPR45/GPR63 ainsi que du sphingosine-1-phosphate et/ou de l'acide lysophosphatidique.
